# EUROPEAN PATENT APPLICATION

(11) **EP 2 196 212 A1**
(43) Date of publication of application: **16.06.2010**
(21) Application number: 08836570.5
(22) Date of filing: 03.10.2008
(51) Int. Cl.: A61K 39/00, A61K 39/39, A61N 1/30, A61P 3/06, A61P 9/12, A61P 25/28, A61P 35/00

(54) **PREPARATION AND METHOD OF ADMINISTERING VACCINE AND IONTOPHORESIS DEVICE USING THE PREPARATION**

(30) Priority: 04.10.2007 JP 2007260534
(71) Applicant: Josai University Corporation, Tokyo 102-0094 (JP)
(72) Inventor: MORI, Kenji, Ryugasaki-city Ibaraki 301-0043 (JP); MIYAGI, Takamitsu, Kisarazu-city Chiba 292-0044 (JP); SHIMADA, Yayoi, Kisarazu-city Chiba 292-0042 (JP); CHIANG, Kuei-Chen, Tokyo 160-0022 (JP); OHMORI, Naoya, Chiba-city Chiba 267-0066 (JP); GOTO, Takeshi, Usiku-city Ibaraki 300-1206 (JP); SATO, Shuji, Narita-city Chiba 286-0018 (JP)
(74) Representative: Fiussello, Francesco
(86) International application number: PCT/JP2008/002783
(87) International publication number: WO 2009/044555

(57) **Abstract**

It is an object of the present invention to provide a method and an apparatus capable of noninvasively administrating vaccine through the skin, and thereby making the immune response activate. A drug product according to the present invention is **characterized in that** the drug product has a support medium, an electrode formed on the support medium and protected partly by an insulating material for avoiding a direct cutaneous contact, a vaccine containing layer for containing a vaccine, wherein the vaccine is an antigen for inducing an immune suppression protein. A method of administrating a vaccine according to the present invention is **characterized in that** the vaccine is administrated by the iontophoresis using the drug product according to the drug product of the present invention as an electrode. An apparatus for the iontophoresis according to the present invention is **characterized in that** the drug product according to the present invention is used as an electrode.

[Sequence listing]
SEQUENCE LISTING

<110> Josai university corporation

<120> drug formulation

<130> PJJI0034

<160> 5

<170> PatentIn version 3.4

<210> 1
<211> 12
<212> PRT
<213> Artificial

<220>
<223> Antigen

<400> 1

Ser Ser Val Leu Tyr Gly Gly Pro Pro Ser Ala Ala
1               5                   10

<210> 2
<211> 12
<212> PRT
<213> Artificial

<220>
<223> Antigen

<400> 2

His Ala Thr Gly Thr His Gly Leu Ser Leu Ser His
1               5                   10

<210> 3
<211> 12
<212> PRT
<213> Artificial

<220>
<223> Antigen

<400> 3

Asn Tyr Gln Thr Tyr Thr Pro Arg Pro Pro His Ser
1               5                   10

<210> 4
<211> 12
<212> PRT
<213> Artificial

<220>
<223> Antigen

<400> 4

Val Thr Asn Asn Gln Thr Ser Pro Arg Trp Glu Ile
1               5                   10

<210> 5
<211> 12

<212>  PRT
<213>  Artificial

<220>
<223>  Antigen

<400>  5

Trp Lys Pro Val Ser Leu Thr Leu His Thr His Pro
1               5                   10

## Description

### Technical Field

The present invention relates to a drug product, a method of administrating a vaccine using the drug product, and an apparatus for iontophoresis.

### Background Art

An iontophoresis method is a method of penetrating an ingredient such as an ionic medical agent which is useful for a living organism, into a living body by using so-called electrophoresis, and the method is also called as an ion transfer therapy, iontophoresis therapy etc., and mainly used for the administration of the systemic medicinal substance.

An apparatus for iontophoresis generally comprises an operation electrode structure for holding a medical agent solution in which a positive ion or a negative ion (a drug ion) are dissociated from medicinal ingredients (physiologically active substance), a non-operation electrode structure for playing a role as a counter electrode of the operation electrode structure, wherein a drug ion may be administrated into the living organism by energizing the same polar voltage as those of the drug ion into the operation electrode structure through the electric power unit under the condition that both structures are abutted on the skin of the living organism (human or mammal (Nonpatent literature 1). There is infrequently an apparatus comprises both electrodes made of the operation electrode structures, and which contain a physiologically active substance. Since the apparatus for iontophoresis generally requires a low current which is less or equal to 1mA per unit area, although there is no painful symptom, a cutaneous reaction resulting in such as a reddish skin may be caused by an electricity.

On the other hand, the vaccine is mainly used for the prevention of an infection disease, and is a pharmacologic product used for the prevention of an infection disease by inoculating it with the animal such as human. The vaccine is generally made from the pathogenic organism which has no toxicity or lower toxicity, and is used for preventing a patient from being liable to the infection disease after administration, by means of the injection of the pathogenic organism to produce an antibody in the body. The vaccine may be divided roughly two type of vaccines, that is, a live vaccine and an inactivated vaccine. Since the live vaccine is made by using a microorganism or a virus having a lower toxicity, and can obtain not only a humoral immune but also a cellular immunity, in general, the live vaccine has a stronger power of the acquired immunity, and a longer duration of the immune effect compared with those of the inactivated vaccine. As those live vaccine, mention may be made of BCG, live oral poliomyelitis vaccine, lymph, measles vaccine, rubella vaccine, measles and rubella combined vaccine, epidemic parotiditis vaccine, yellow fever vaccine, rotavirus vaccine, zoster vaccine, measles - rubella - mumps combined vaccine, MMRV (measles, rubella, mumps, varicella) etc.

On the other hand, an inactivated vaccine is also called a dead vaccine. As a narrowly-defined inactivated vaccine, virus, bacteria and rickettsia are used which are dead by the chemical treatment etc. Those which only a antigenic part is cultured are also called an inactivated vaccine, since they have the same effects. Although the inactivated vaccine has a lower side reaction than the live vaccine, it can obtain only a humoral immune, and thereby the live vaccine having a shorter duration of the immune effect compared with those of the live vaccine. And therefore, in a lot of cases, they need one or more inoculations. As an example of these inactivated vaccine, mention may be made of influenza virus vaccines, rabies vaccine, cholera vaccine, diphtheria pertussis tetanus (DPT) vaccine (pertussis, diphtheria, tetanus combined vaccine), diphtheria tetanus (DT) vaccine (diphtheria, tetanus combined vaccine), Japanese encephalitis vaccine, pertussis vaccine, 23 valent pneumococcal capsular polysaccharide vaccine, hepatitis type A virus vaccine, hepatitis type B virus vaccine, polyvalent binding protein pneumococcal vaccine, anthrax vaccine, inactivated poliovirus vaccine, meningococcus vaccine, typhoid vaccine, tick-borne encephalitides vaccine, influenza bacillus type b vaccine, hepatitis type A vaccine, diphtheria and tetanus combined vaccine, new type of an inactivated oral cholera vaccine, human papillomavirus (HPV) vaccine, etc.

Moreover, the combination of the antigen with the other proteins comes under review, because it may be impossible to confer immunity with the use of only a part of antigen in the case of a vaccine in which an antigen other than the protein such as pneumococcal vaccine is used, or in the case of an antigen which has a low immunity activity.

Recently, the vaccine begin to have a much broader range of applications compared with the previous concept that the vaccine is designed for the prevention toward the foreign pathogenesis factor such as virus, bacteria etc., according to the development of the vaccine with the use of new administration route which is different from the injection, such as oral vaccine, percutaneous vaccine, vaginal vaccine, transrectal vaccine, transnasal vaccine, lung vaccine (vaccine through the lung). And the vaccine begins to be developed for the medical treatment for a prevention of the chronicity of the affected illness or a noninfectious disease, for example auto immune disease such as disseminated sclerosis or myasthenia gravis, cancer, dementia, elevated blood pressure, hyperlipemia.

As an inoculation method of the vaccine, there is an oral administration like a polio. Further, BCG is known as a stamp type of an administration method wherein it comprises several pieces of needle, and is also known as an inoculation method wherein it relatively renders a lower pain.

Furthermore, as an inoculation method of the vaccine with the use of iontophoresis, it is known an example comprises one electrode of the iontophoresis including a vaccine, the other electrode including an adjuvant for increasing an immunity activity (Patent literature 1)
Patent literature 1 : JP-A-2006-334292
Nonpatent literature 1 : Journal of pharmaceutical science, Vol. 76, page 341, 1987

### Disclosure of the Invention

### Problems to be resolved by the invention

However, as the inoculation method of vaccine, in general, most of them are administration methods by an invasive injection other than the oral administration method according to the above polio. It is not convenient means as the administration method of vaccine which needs several administrations, because most of them other than the polio as mentioned above are an invasive administration method, they not only involve a risk of infection etc., but also absolutely need an administration in the medical institution. Further, in advancing nations, there is a problem of the infection caused by the recycle of an inadequately - sterilized injection syringe or injection needle in the administration of vaccine be means of the injection. On the other hand, in advanced nation, such as the United States which actively advances the development of vaccine, there is a problem that it is impossible to promptly carry out the large scale administration of vaccine since the administration by the injection requires the skilled job when a bioterrorism occurs, or a fatal infection such as a highly toxic influenza or yellow fever occur in epidemics. Furthermore, in both advancing nations and advanced nations, there is a common problem of the infection caused by a needle stick accident of the medical staff, and the establishment of a convenient administration method of vaccine compared with the administration by the injection is desired. Therefore, a noninvasive and effective administration method of vaccine have been made researches with the use of the immunity of skin as a new administration method of vaccine in substitution for the administration by injection.

However, in the present circumstances, it is difficult to make the vaccine transmit into the stratum corneum, and thereby to activate the immune response by the mere paste of vaccine, since the skin make a most outer shell, and covered with the stratum corneum having an ability of barrier. Moreover, there is a problem that it is not easy to activate the immune response, even if the vaccine is administrated into a skin from which the stratum corneum is removed, so called stripped skin.

Furthermore, in the above patent literature 1, although it is disclosed an art for administrating vaccine according to the iontophoresis, it absolutely needs the use of adjuvant because it is impossible to make the immune response activate effectively, by means of the use of only an antigen. However, since there is a risk of side effects deriving from the adjuvant in the case of the use of adjuvant, if possible, an administration of vaccine with no use of adjuvant is desired. However, at this moment, there are no administration method of vaccine using the iontophoresis capable of effectively activating the immune response with no use of adjuvant.

Therefore, it is an object of the present invention to provide a method capable of noninvasively administrating vaccine through the skin, and thereby making the immune response activate within an organism by using the immunity of skin with no use of adjuvant.

### Means of solving the problems

In order to accomplish the above objects, the present inventors made strenuous studies on a noninvasive administration of vaccine. As a result, the inventors discovered the present invention.

That is, a drug product according to the present invention is **characterized in that** the drug product has a support medium, an electrode formed on the support medium and protected partly by an insulating material for avoiding a direct cutaneous contact, a vaccine containing layer for containing a vaccine, wherein the vaccine is an antigen for inducing an immune suppression protein.

Furthermore, in a preferred embodiment of a drug product according to the present invention, the drug product is **characterized in that** the antigen for inducing an immune suppression protein is a histone H1, or a peptide described in any one of the sequence numbers 1 to 5 of the sequence listing.

Furthermore, in a preferred embodiment of a drug product according to the present invention, the drug product is **characterized in that** a pH of a drug solution existing in the vaccine containing layer is greater than or equal to 6.

Furthermore, in a preferred embodiment of a drug product according to the present invention, the drug product is **characterized in that** the vaccine contains a carrier protein.

Furthermore, in a preferred embodiment of a drug product according to the present invention, the drug product is **characterized in that** the carrier protein is animal protein, plant protein or glycoprotein.

Furthermore, in a preferred embodiment of a drug product according to the present invention, the drug product is **characterized in that** the carrier protein is at least one selected from the group comprising keyhole limpet hemocyanin (KLH), ovalbumin (OVA), bovine serum albumin (BSA).

Furthermore, in a preferred embodiment of a drug product according to the present invention, the drug product is **characterized in that** the vaccine contains at least one adjuvant selected from the group comprising aluminum compound, w/o type of emulsion, o/w type of emulsion, liposome, virosome, saponin, saponin inclusion, MF59 (squalene emulsion), monophosphoryl lipid A (MPL), heat-labile enterotoxin, cholera toxin, CpG motif oligonucleotide, cytokine, nonionic block copolymer.

Furthermore, in a preferred embodiment of a drug product according to the present invention, the drug product is **characterized in that** the vaccine is an inactivated vaccine.

A method of administrating a vaccine according to the present invention is **characterized in that** the vaccine is administrated by the iontophoresis using the drug product according to the drug product of the present invention as an electrode.

Furthermore, in a preferred embodiment of a method of administrating a vaccine according to the present invention, the method is **characterized in that** an immunity of the skin is induced by making an immune signal of the skin generate when administrating the vaccine.

Furthermore, in a preferred embodiment of a method of administrating a vaccine according to the present invention, the method is
**characterized in that** an induction of the immunity of the skin is further carried out by the electric stimulus.

Furthermore, in a preferred embodiment of a method of administrating a vaccine according to the present invention, the method is **characterized in that** the first administration of the vaccine is carried out under the condition that a pH of a drug solution existing in the vaccine containing layer is greater than or equal to 6.

Furthermore, in a preferred embodiment of a method of administrating a vaccine according to the present invention, the method is **characterized in that** the first administration of the vaccine is carried out under the condition that a pH of a drug solution existing in the vaccine containing layer is greater than or equal to 6, followed by the second or later administration of the vaccine is further carried out.

An apparatus for iontophoresis according to the present invention is **characterized in that** the drug product according to the present invention is used as an electrode.

Furthermore, in a preferred embodiment of an apparatus for iontophoresis according to the present invention, the apparatus for iontophoresis is **characterized in that** the drug product is used as an electrode of the cathode side.

Furthermore, in a preferred embodiment of an apparatus for iontophoresis according to the present invention, the apparatus for iontophoresis is **characterized in that** the drug product is used as an electrode of both the cathode side and the anode side.

### Effect of Invention

The drug product, the method of administrating vaccine, and the apparatus for iontophoresis according to the present invention have advantageous effects as mentioned below.

(1) Although a lot of the administration of vaccine are particularly carried out at the time of baby, the administration according to the apparatus of the present invention makes it possible to acquire the immunity without causing a pain to baby or child who can not live with a pain.

(2) It is possible to improve an compliance for patients having a fear to the needle that an administration of vaccine by injection is difficult.

(3) It is possible to avoid the risk of the infection disease caused by a needle stick accident of the medical staff occurring in the administration by injection.

(4) It is possible to administrate conveniently and easily induce the immune response since it is not accompanied by pain even if a vaccine including an antigen needed for one or more administration because of a lower immunogenicity is used.

(5) It is possible to prevent the infection from the skin by bacteria etc., because it is noninvasive.

(6) It can obtain effects, for example, that it is possible to administrate the vaccine in home, and to prevent the disease in home because it require only pasting a patch containing vaccine to the skin, and applying a voltage from the predetermined apparatus.

(7) The applications of the iontophoresis technique to the administration of vaccine, which was mainly used for beautification or treatment of disease in the past, make it possible to induce an immune response through a percutaneous route, and prevent the occurrence of disease.

(8) The iontophoresis technique makes it possible to reduce a dosage of antigen compared with the other administration route because an existence of a great deal of the Langerhans cell which is a target cell and is located near the skin of the site of administrating an antigen, makes it possible to effectively transport the antigen into the target cell. Further, it has an advantageous effect that it is possible to reduce a cost because a cold chain during the production, the transportation and the consumption can be unnecessary which was required in a vaccine for the administration of injection, and to administrate a vaccine on a large scale according to the improvement of convenient means for the administration.

As mentioned above, the present inventions made it possible to enhance an ability for the immune response of vaccine by means of the administration of vaccine through the skin according to the iontophoresis. Further, it became possible to noninvasively carry out the administration of vaccine.

### Brief Description of the Drawings

[Figure 1] Fig. 1 gives a figure showing a gross outline of the apparatus for iontophoresis as an example of an embodiment according to the present invention.
[Figure 2] Fig. 2 gives a figure showing a drug product as an example of an embodiment according to the present invention.
[Figure 3] Fig. 3 gives a average value ± standard error of the absorbance of serum samples at the time of 25 days after the beginning of the test of each group.

### Best Mode for Carrying Out the Invention

A drug product according to the present invention is a drug product characterized by having a support medium, an electrode formed on the support medium and protected partly by an insulating material for avoiding a direct cutaneous contact, a vaccine containing layer for containing a vaccine, wherein the vaccine is an antigen for inducing an immune suppression protein.

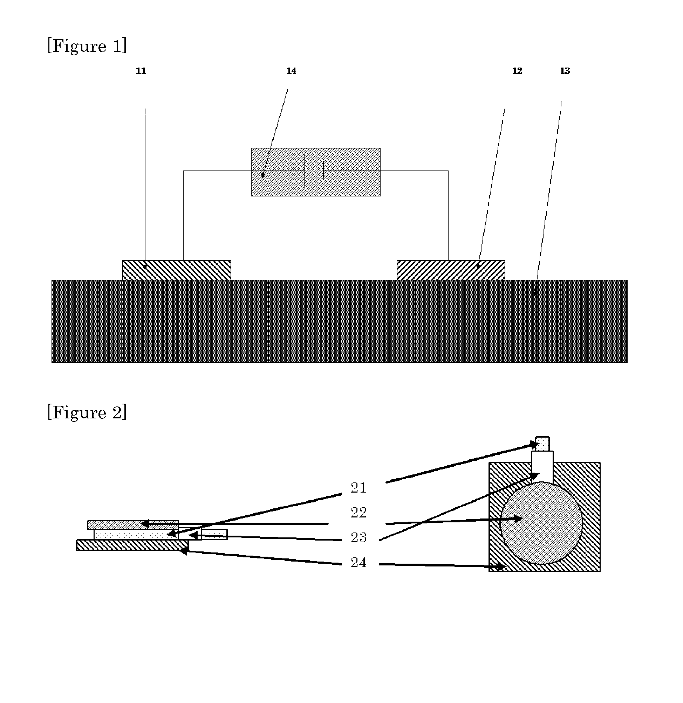

At this moment, the explanation of an example of the drug product according to the present invention using Figure 2 is as follows. By reference to Figure 2, the drug product according to the present invention having a support medium (24), an electrode (21) formed on the support medium and protected partly by an insulating material (23) in order to avoid a direct cutaneous contact, a vaccine containing layer (22) for containing a vaccine. For example, an electrode (21) protected partly by an insulating tape (23) in order to avoid a direct cutaneous contact between a terminal area and the skin, may be fixed with an adhesive material etc., on a support medium (24) comprising a synthetic polymer film such as a PET film or a foam, and a vaccine containing layer (22) may be laminated on the electrode. The support medium is not particularly limited, as long as it can play a role in fixing it with the skin. A vaccine containing layer is not particularly limited to any materials etc., as long as it is possible to hold a solution, or a drug solution for containing vaccine. For example, a nonwoven fabric etc., may be utilized for the vaccine containing layer. Further, an insulating material is not particularly limited, as long as it can play a role in avoiding a direct contact with the skin. And the constitution of the insulating material may be a structure in which a part of the electrode is surrounded by the insulating material, is not particularly limited, as long as it can avoid a direct contact depending on the pattern of a connection with the devices. Therefore, it is not necessarily to cover all part of the electrode with the insulating material, a part of the electrode may be protected by the insulating material. An electrically conductive lead etc., may be connected to a part of running off the edge of the electrode in Figure 2. In a preferred embodiment, as a material of the electrode, at lease one type may be selected from an electrode comprising the electron conductive materials such as platinum, gold, titanium, aluminum, carbon, silver, copper, nickel, zinc, carbon, an semiconductor electrode, a self - sacrifice electrode such as silver / silver chloride, silver chloride / silver chloride. Carbon is preferable as the electrode from a viewpoint that it is possible to utilize it at low cost. Further preferably, those containing copper at an anode side, and those containing silver chloride at a cathode side are desired from a viewpoint that there is no change of pH value on the issue of the function. However, the present invention is not limited to these one. These electrode may also be used as is in the conditions that a board, a sheet, a mesh, a fabric etc., are molded to obtain an undefined laminated material like a paper.

At this moment, although the term "vaccine" had been mainly used for the prevention of the infection disease in the past, the term "vaccine" used herein means more broad concept. That is, in the present invention, the term "vaccine" means a broad concept including not only those for an infection disease, but also those for the medical treatment for a prevention of the chronicity of the affected illness or a noninfectious disease, for example auto immune disease such as disseminated sclerosis or myasthenia gravis, cancer, dementia, elevated blood pressure, hyperlipemia.

This is because, recently, the vaccine begin to have a much broader range of applications compared with the previous concept that the vaccine is designed for the prevention toward the foreign pathogenesis factor such as virus, bacteria etc., according to the development of the vaccine with the use of new administration route which is different from the injection, such as oral vaccine, percutaneous vaccine, vaginal vaccine, transrectal vaccine, transnasal vaccine, lung vaccine (vaccine through the lung). And the vaccine begins to be developed for the medical treatment for a prevention of the chronicity of the affected illness or a noninfectious disease, for example auto immune disease such as disseminated sclerosis or myasthenia gravis, cancer, dementia, elevated blood pressure, hyperlipemia, and therefore the present invention may have applicability to a broad range of area regardless of the possibility of the infection or noninfection.

The vaccine containing layer (a retentive portion of the component) is also not limited, but as a retentive portion of the component, mention may be made of those of impregnating a vaccine containing solution in which all or a part of a vaccine are dissolved in a water, into a porous polymer or a gauze, or those like a gel obtained by mixing the vaccine containing solution with polyvinyl alcohol or polyvinyl pyrrolidone.

Moreover, in a preferred embodiment of the drug product according to the present invention, the antigen for inducing an immune suppression protein may be a histone H1 as a protein. At this moment, providing an explanation of a histone, a histone means a group of proteins which constitute a chromatin of an eucaryotic organism. The histone is a strong basic protein, and has a high degree of affinity for an acidic DNA. The histone play a role in allowing for compact storage of DNA by winding itself around the histone. It is known five different type of the histones(H1, H2A, H2B, H3, H4). Among them, fore type, that is, H2A, H2B, H3, H4 is called as core histone, a histone octamer is formed by aggregating a bimolecular of each core histone. One histone octamer can allow about 146bp (base pairs) of DNA to sinistrorsely wind itself around the histone octamer in about 1.65 times. This structure is so called as the "nucleosome", and is the minimum unit of a structure of the chromatin. H1 is so called as a linker histone and allowing the binding to the DNA existing in between the nucleosomes.

Moreover, the antigen for inducing an immune suppression protein may be a peptide described in any one of the sequence numbers 1 to 5 of the sequence listing, specifically, mention may be made of SSVLYGGPPSAA, HATGTHGLSLSH, NYQTYTPRPPHS, VTNNQTSPRWEI, WKPVSLTLHTHP etc.

In a preferred embodiment, a pH of a drug solution existing in the vaccine containing layer is greater than or equal to 6. Although a detailed mechanism about this is unclear, it is thought that the treatment of the skin with the pH being greater than or equal to 6, preferably the treatment with the pH of alkali, more preferably the treatment with the pH of 7.5 to 9.5 makes it possible to generate a skin injury signal and thereby causing an induction of the immunity of the skin. Further, the use of the drug product according to the present invention has a better than expected vaccine effect through a synergy effect with the induction of the immunity of the skin according to the electric stimulus caused by the iontophoresis.

Furthermore, in a preferred embodiment of a drug product according to the present invention, the vaccine contains a carrier protein. A carrier protein is not particularly limited, but mention may be made of animal protein, plant protein or glycoprotein and or the like. For example, As the carrier protein, mention may be made of at least one selected from the group comprising keyhole limpet hemocyanin (KLH), ovalbumin (OVA), bovine serum albumin (BSA). A coupling style between the vaccine and the carrier protein is not particularly limited, but it may depend on the conventional procedures. It is generally known a method wherein in the case of an antigen having a low immunogenicity (like an antigen which can not induce an antibody by itself), an antigen is coupled with a protein having a high immunogenicity so called a carrier protein, and thereby inducing an antibody for the carrier protein as well as inducing an antibody for a target antigen at the same time. Therefore, it may have applicability to the drug product of the present invention. Although there are so many number of the coupling styles between the carrier protein and the antigen, they are preferably coupled with each other by a covalent bind from the viewpoint that it is possible to efficiently convey an antigen to the antigen presenting cells. It may be coupled through the intermediary of glutaraldehyde etc.

Furthermore, the vaccine may contain at least one adjuvant selected from the group comprising aluminum compound, w/o type of emulsion, o/w type of emulsion, liposome, virosome, saponin, saponin inclusion, MF59 (squalene emulsion), monophosphoryl lipid A (MPL), heat-labile enterotoxin, cholera toxin, CpG motif oligonucleotide, cytokine, nonionic block copolymer.

Next, the explanation of the method of administrating a vaccine according to the present invention is as follows. That is, the method of administrating a vaccine according to the present invention is **characterized in that** the vaccine is administrated by the iontophoresis using the drug product according to the present invention as an electrode. This is because that the present inventors found out that the administration of the vaccine by the iontophoresis using the drug product according to the present invention as mentioned above makes it possible to administrate the vaccine without the use of the adjuvant. Concerning the drug product of the present invention used for the method of administration according to the present invention, the above mentioned explanation of the drug product of the present invention can be applicable to the method of administrating the vaccine according to the present invention. It is possible to noninvasively administrate the vaccine by the iontophoresis, and thereby acquiring an immunity.

Moreover, an apparatus for according to the present invention is **characterized in that** the drug product according to the present invention is used as an electrode. Concerning the drug product of the present invention used for the apparatus for iontophoresis according to the present invention, the above mentioned explanation of the drug product of the present invention can be applicable to the apparatus for iontophoresis according to the present invention.

In a preferred embodiment according to the present invention, an immunity of the skin can be induced by making an immune signal of the skin generate when administrating the vaccine. A procedure for inducing the immunity of the skin by means of making an immune signal of the skin generate is not particularly limited. For example, it is possible to induce the immunity of the skin with the use of the drug product of the present invention under the condition that a pH of a drug solution existing in the vaccine containing layer is greater than or equal to 6.

Moreover, in a preferred embodiment of a method of administrating a vaccine according to the present invention, an induction of the immunity of the skin may be further carried out by the electric stimulus. The electric stimulus, for example, the induction of the immunity of the skin with use of the iontophoresis makes it possible to attain a better than expected vaccine effect.

Furthermore, in a preferred embodiment of a method of administrating a vaccine according to the present invention, from the viewpoint of the induction of the immunity of the skin, the first administration of the vaccine is carried out under the condition that a pH of a drug solution existing in the vaccine containing layer is greater than or equal to 6. In general, the first administration is commonly called as a priming (the first stimulus), the second or later administration is called as a boost.

Moreover, in a preferred embodiment, the first administration of the vaccine is carried out under the condition that a pH of a drug solution existing in the vaccine containing layer is greater than or equal to 6, followed by the second or later administration of the vaccine is further carried out. Moreover, terms and conditions such as a drug solution etc., of the second or later administration of the vaccine is not particularly limited. For example, only an antigen etc., may be administrated. Further, an antigen together with the carrier protein as mentioned above or an existing adjuvant may be administrated. An antigen together with both the carrier protein and an existing adjuvant may be administrated.

At this moment, the brief explanation of the iontophoresis as an example is as follows. The iontophoresis means those capable of penetrating an ionic component valuable for a living body into the living body with the use of the electrophoresis. Figure 1 shows an example of the administration. An anodic electrode (11) and a cathode electrode (12) are pasted on the skin (13), to the electrodes are applied the electric current by energizing from the electric power unit (14). At this point, if a physiologically active substance is electrostatically and positively charged, it may be included in the anode side, and if a physiologically active substance is negatively charged, it may be included in the cathode side. An amphoteric compound comprising an amino acid or a protein like vaccine may be positively charged or negatively charged by means of on the adjustment of pH of the solution for solving it, and thereby being able to contain it in the anode side or the cathode side, alternatively both the anode and cathode side.

Regarding the application of the drug product of the present invention as mentioned above as an electrode, for example, a drug product for the iontophoresis containing the vaccine may be applied on the skin as an anode electrode or a cathode electrode, alternatively both electrode, as illustrated by the Figure 1.

Moreover, since the vaccine is generally a high polymer compound, it has low proportion of the contribution for the electrically migration (Low transportation value) compared with those of a low-molecular-weight compound such as Na⁺, Ag⁺, Cl⁻, H⁺, OH⁻. Therefore, an ion exchange membrane or an ion exchange resin may be used in between the electrode and the vaccine containing layer to prevent the vaccine from being mixed with the ions (Ag⁺, Cl⁻, H⁺, OH⁻, etc.) generated by turning on electricity from the electrode, and to prevent the degradation of the transportation value for the vaccine. Furthermore, although a counter ion of the living body abstracted from the living body side (which is an ion existing in the living body, and is electrostatically charged in an opposite conductivity type compared with those of a component of an ion which should be introduced), in particular, a counter ion having a higher mobility (for example, Na⁺ or Cl⁻ etc.) is mainly released, an ion exchange membrane or an ion exchange resin may be used in between the skin and the vaccine containing layer to administrate the vaccine, in order to prevent the counter ion from being released and as well as to enhance the transportation value.

As mentioned above, an administration of the vaccine according to the iontophoresis with the use of the drug product of the present invention makes it possible to administrate the vaccine with no use of adjuvant. The reasons why it is possible to administrate the vaccine with no use of the adjuvant is, as an example, because an external stimulus according to the drug product of the present invention is comparable to an adjuvant effect. That is, in general, there is a problem that in the case of a percutaneous immunity compared with the other route of administration, an activity of the immunity induction is low, and therefore, it is necessary to use the adjuvant. On the other hand, it is thought that in the case of the use of the iontophoresis, the electric stimulation becomes a signal for external hazard, and then plays a role in the activation of the immunity. The adjuvant may be widely classified into 5 types. It is supposed that among them, the electric stimulation gives an adjuvant effect corresponding to the external stimulus, danger signal (for example, an adjustment of the medicinal agent to the alkaline side, as mentioned above).

That is, according to the present invention, it is possible to give the external stimulus, for example, which can be caused by an adjustment of the pH of a solution containing the medicinal agent to the alkali side, in order to make a skin injury signal generate and to cause an induction of the immunity of the skin, and thereby attaining a better than expected vaccine effect through a synergy effect with the induction of the immunity of the skin according to the electric stimulus caused by the iontophoresis if the adjuvant is used.

### Example

The present invention will be concretely explained in more detail with reference to Examples, but the present invention is not intended to be interpreted as being limited to Examples.

### Example 1

At first, in the case that the vaccine is applied with the iontophoresis, he effect of the present invention was examined.

### <Administration, Blood collection>

A dorsal region and an abdominal region of a mouse (Balb/c mouse, age in 4 weeks, female) were carefully dehaired by a hair clipper so as not to cause damage, and further the site of administration for the vaccine was entirely dehaired with the use of the hair remover which is commercially produced (Epilat: Kanebo). As shown in Figure 2, a part of the silver / silver chloride electrode (21) is protected by the insulating tape (23), and these were laminated on the support medium (24). Further, a bonded textile (22, 3.14 cm² of area) was laminated on the electrode. A 200 µL of the vaccine solution shown in formulation 1 was applied to a part of the bonded textile of the drug product, and this was used as an anode electrode. Moreover, a 200 µL of the vaccine solution shown in formulation 2 was applied to the drug product of Figure 2, and this was used as a cathode electrode. The anode electrode and the cathode electrode were pasted in the abdominal region and the dorsal region respectively, followed by to this being turned on electricity at constant current, 1.57 mA for 30 minutes through the electric power unit (V1002: Furesaisugeji). At 1 week after the administration of the vaccine, the blood samples were collected from the caudal vein, and were separated by the centrifugation to obtain a blood serum. An anti-SSV antibody existing in the blood serum was examined by the ELISA (Enzyme-linked immunosorbent assay) method. The administration, the collecting blood samples and the measurement are defined as one cycle, the experimentation was carried out for 4 weeks at a frequency of one cycle every 2 weeks.

### <Determinate quantity>

An amount of the antibody formation was examined by the ELISA method. Each blood samples were collected from each mice, and then an amount of the antibody existing in the mouse blood serum was examined by the ELISA method according to the following procedure. Moreover, as hereinafter defined, an OVA-SSV means a complex comprising an ovalbumin and a peptide having an amino acid sequence shown in the sequence number 1. The OVA-SSV was prepared synthetically by the same manner as those of a complex comprising a peptide and KLH.

That is, in the case that the antigenic substance is prepared artificially, for example, it is possible to use a known art of synthesizing peptide such as a solid phase peptide synthesis method, a liquid phase peptide synthesis method etc. Moreover, a method for coupling an antigenic substance with a carrier is not particularly limited as long as it does not prevent the immunogenicity of the antigenic substance, but mention may be made of a method for coupling an antigenic substance with a carrier using a dehydration - condensation agent such as EDC (Ethylendichloride), DCC (dicyclohexyl carbodiimide), DIC (1,3 - diisopropyl carbodiimide), a cross-linker such as glutaraldehyde, maleimide, maleimide benzoyl oxysuccinic acid, a linker such as PEG, linker peptide etc. The antigenic substance may be coupled with the above carrier through the intermediary of carbodiimide or glutaraldehyde. As a manufacturing procedure about the coupling between the peptide and the carrier, for example, mention may be made of the method described in "Experimental basis for peptide synthesis" Nobuo Izumiya et al, Maruzen Company, Limited). The antigenic substance was made synthetically according to the common procedure like this.

A peptide was prepared as the following manner.

### <Identification of recognition site of an antihistone H1 monoclonal antibody>

### <Phage >

A panning examination was carried out about an antihistone H1 antibody produced from hybridomas 1F5, 3F2, 15F11, 17C2 or 16G9 with the use of a Ph. D. -12 Phage display peptide library kit (which is available from New England BioLabs, Inc.). Purified each monoclonal antibodies were solved in 0.1 M NaHCO₃ (pH 8.6), and directly coated on the microtiter plate (Nunc, catalog #430341) to incubate it at 4 °C all night. To each well was added blocking buffer (0.1 M NaHCO₃, 5mg/ml BSA, 0.02%NaN₃), to incubate it for at least 1 hour at 4 °C. After that, those were washed with TBST (50mM Tris, 150mM NaCl, 0.1%Tween 20). In the first panning, 4 × 10¹⁰ of phage existing in the original library were used for screening. A phage to which could not become attached, was removed by being washed repeatedly with TBST. A phage to which could become attached, was eluted by 0.2 M Glycine - HCl (pH 2.2), 1mg/ml BSA. The eluted phages were proliferated with the use of the 20mL of E coli ER2738 culture. The phages obtained thus were precipitated with the use of polyethylene glycol, and used for the second panning. The third panning was carried out according to the same operating procedure. A plaque obtained in the third panning was diluented by 1 : 100, was proliferated with the use of ER2738 culture. A tube containing these plaques was incubated at 37 °C with shaking for 4.5 to 5 hours. A single - strand DNA was precipitated and purified with Iodide buffer (Iodide buffer : 10mM Tris - HCl, 1mM EDTA, 4M Nal) and ethanol. A phage DNA was dissolved in 20 µL TE buffer (10mM Tris = HCl pH8.0, 1mM EDTA) for the analysis of the DNA sequence.

### <The analysis of the DNA sequence>

The sequencing PCR reaction was carried out about the purified phage obtain thus with the use of a primer DNA attached in the above Ph. D. - 12 phage display peptide library kit and a DYEnamic™ ET Terminator Cycle Sequencing Premix Kit (Amercham Biosciences) (PCR reaction conditions : at 95 °C (30 second), next at 50 °C (15 second), and then at 60 °C (1 minute), for 30 cycles). The PCR products were purified by using the AutoSeq™ G-50 (Amersham Biosciences). And then, the DNA sequence of the phage peptide was determined by the ABIPRISM™ 310 Genetic Analyzer (PE Biosystems). The amino acid sequences based on the determined DNA sequences were shown as follows.

### <The hybridoma strain, amino acid sequence>

1F5 : NYQTYTPRPPHS (The sequence number 3 of the sequence table)
3 F 2 : VTNNQTSPRWEI (The sequence number 4 of the sequence table)
15 F 11 : WKPVSLTLHTHP (The sequence number 5 of the sequence table)
17C2 : HATGTHGLSLSH (The sequence number 2 of the sequence table)
16G9 : SSVLYGGPPSAA (The sequence number 1 of the sequence table)

### <The competition ELISA>

Each peptides having the amino acid sequences determined by the above phage DNA were prepared synthetically according to the common procedure. The competition ELISA was carried out by using the peptides obtained thus, the purified each monoclonal antibodies and the histone H1 antigen (Roche, catalog #1004875). In this case, the histone H1 antigen was biotinylated by using the EZ - Link Sulfo - NHS - Biothinylation Kit (Pierce), and the ABTS solution (Sigma, A3219) was used as the coloring reagent. The color was detected by using the ELISA measuring device (ThermoLabsystem, Multiskan Ascent) at 405 nm. The measurement value was calculated at an average rate of 3 times of the absorption value.

As a result of this, it was confirmed that the above peptide prepared synthetically thus can inhibit the bond between the purified each monoclonal antibody and the histone H1 antigen.

At first, a histone H1 solution (20 µg/mL, Roche) or an OVA - SSV solution (OVA - SSV: 0.387 mg/mL, solvent : 0.02 M phosphate buffer solution, 0.9% NaCl, pH 8.0) were prepared by using a 0.1 M NaHCO₃ (pH9.3) solution. Next, 50µL of the solution obtained thus was added into each wells of the 96 holes plate and allowed at room temperature for 1 hour. Next, after each wells were washed three times by the PBST, 150 µL of the PBS solution (3% milk, 1% BSA containing PBS solution) was added into each wells to incubate at 37 °C for 1 hour. Next, after each wells were washed three times by the PBST, 50 µL of the mouse blood serum diluted thousandfold, was added into the wells and allowed at room temperature for 1 hour. Next, after each wells were washed three times by the PBST, 50 µL of the mouse IgG labeled by peroxidase (SIGMA) and diluted 2000 to 4000 folds with the PBST, was added the wells, and allowed at room temperature for 1 hour. Next, after each wells were washed three times by the PBST, the ABTS (2, 2' -azino - bis [3 - ethylbenzoline - 6 - sulfonate], SIBMA) was added as a coloring substrate to incubate for 30 to 60 minutes. After that, the absorbance of each wells were determined by Multiscan Ascent (Thermo Labsystems, 405 nm of the wave length).

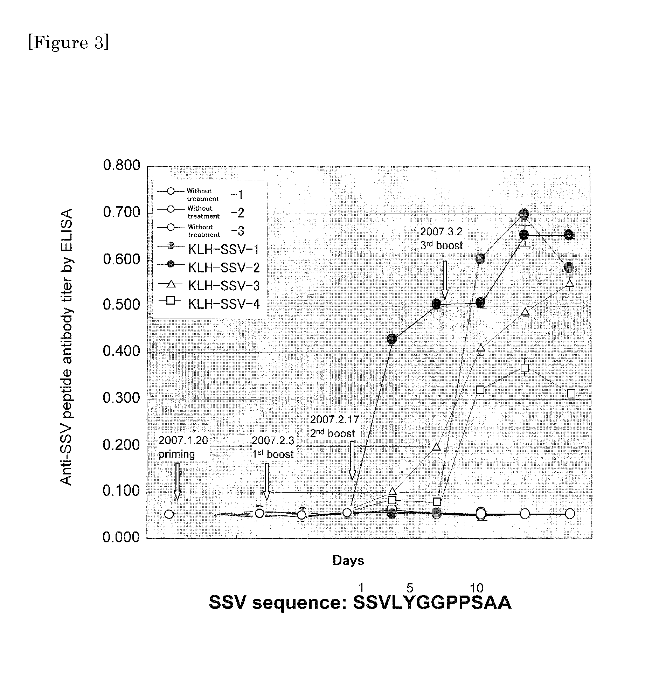

The result of this, an average value ± standard error of the absorbance of the blood serum samples at 25 days after the beginning of the test about each groups were shown in Figure 3.

The average value ± standard error of the absorbance of the measurement samples was 0.524 ± 0.146 in the case of the use of a complex of an antigen peptide for the immunologic tolerance induction vaccine and the KLH. It was confirmed that in the case that a complex of an antigen peptide for the immunologic tolerance induction vaccine and the KLH were used and further the administration was carried out by the iontophoresis, a production quantity of the antibody was higher compared with those in the case of only pasting of a vaccine solution having the same composition

The result mentioned above, it was revealed that a synergy effect made by both the induction of the immunity of the skin according to the electric stimulus caused by the iontophoresis and the induction of the immunity of the skin according to the skin injury signal gives a better than expected vaccine effect. Further, it was also revealed that The Langerhans cell was activated by the alkali treatment of the skin + the use of the iontophoresis method.

### Comparative example 1

Next, the effects in the case of only pasting of a vaccine solution having the same composition as the Example 1 were examined.

### <Administration>

A dorsal region and an abdominal region of a mouse (Balb/c mouse, age in 4 weeks, female) were carefully dehaired by a hair clipper so as not to cause damage, and further the site of administration for the vaccine was entirely dehaired with the use of the hair remover which is commercially produced (Epilat: Kanebo). A 200 µL of the vaccine solution shown in formulation 1 was applied to a part of the bonded textile of the drug product comprising the silver / silver chloride electrode, a support medium, a bonded textile (3.14 cm² of area). The drug product was pasted on the dorsal region and the abdominal region. At 1 week after the administration of the vaccine, the blood samples were collected from the caudal vein, and were separated by the centrifugation to obtain a blood serum. The anti - histone H1 antibody, the anti - SSV antibody, or the anti - LPQ antibody existing in the blood serum was examined by the ELISA (Enzyme-linked immunosorbent assay) method. The administration, the collecting blood samples and the measurement are defined as one cycle, the experimentation was carried out for 6 to 10 weeks at a frequency of one cycle every 2 weeks.

### <Quantitative determination>

The experimentation was carried out by the ELISA.

As a result of this, it was revealed that there are no effects of the administration of the vaccine if those of the same composition as the Example 1 are merely pasted.

### Comparative example 2

Next, the effects in the case of the application of those of the same composition as Example 1 for a tape stripping mouse were examined.

### <Administration>

A dorsal region and an abdominal region of a mouse (Balb/c mouse, age in 4 weeks, female) were carefully dehaired by a hair clipper so as not to cause damage, and further the site of administration for the vaccine was entirely dehaired with the use of the hair remover which is commercially produced (Epilat: Kanebo). An adhesive cellophane tape were repeatedly pasted and delaminated 20 times to remove a stratum corneum, and thereby obtaining a stripped skin. The same drug product as Comparative Example 1 was pasted on the stripped skin. At 1 week after the administration of the vaccine, the blood samples were collected from the caudal vein, and were separated by the centrifugation to obtain a blood serum. The anti - SSV antibody existing in the blood serum was examined by the ELISA method. The administration, the collecting blood samples and the measurement are defined as one cycle, the experimentation was carried out for 6 weeks at a frequency of one cycle every 2 weeks.

### <Quantitative determination>

The experimentation was carried out by the ELISA.

As a result of this, it was revealed that there are no effects of the administration of the vaccine if those of the same composition as the Example 1 are merely pasted.

### Industrial applicability

The drug product, a method of administrating a vaccine using the drug Product, and an apparatus for iontophoresis according to the present inventions make a significant contribution in producing a research guide of the broad fields such as biological chemistry, biochemistry, medical science, pharmaceutical sciences since those are noninvasive, safe, easy-to-use and effective.

## Claims

1. A drug product having a support medium, an electrode formed on the support medium and protected partly by an insulating material for avoiding a direct cutaneous contact, a vaccine containing layer for containing a vaccine, wherein the vaccine is an antigen for inducing an immune suppression protein.

2. A drug product according to claim 1, wherein the antigen for inducing an immune suppression protein is a histone H1, or a peptide described in any one of the sequence numbers 1 to 5 of the sequence listing.

3. A drug product according to claims 1 or 2, wherein a pH of a drug solution existing in the vaccine containing layer is greater than or equal to 6.

4. A drug product according to any one of claims 1 to 3, wherein the vaccine contains a carrier protein.

5. A drug product according to claim 4, wherein the carrier protein is animal protein, plant protein or glycoprotein.

6. A drug product according to any one of claims 1 to 4, wherein the carrier protein is at least one selected from the group comprising keyhole limpet hemocyanin (KLH), ovalbumin (OVA), bovine serum albumin (BSA).

7. A drug product according to any one of claims 1 to 6, wherein the vaccine contains at least one adjuvant selected from the group comprising aluminum compound, w/o type of emulsion, o/w type of emulsion, liposome, virosome, saponin, saponin inclusion, MF59 (squalene emulsion), monophosphoryl lipid A (MPL), heat-labile enterotoxin, cholera toxin, CpG motif oligonucleotide, cytokine, nonionic block copolymer.

8. A drug product according to any one of claims 1 to 7, wherein the vaccine is a vaccine containing an antigen for inducing an immune suppression protein.

9. A method of administrating a vaccine by the iontophoresis using the drug product according to any one of claims 1 to 8 as an electrode.

10. A method of administrating a vaccine according to claim 9, wherein an immunity of the skin is induced by making an immune signal of the skin generate when administrating the vaccine.

11. A method of administrating a vaccine according to claims 9 or 10, wherein an induction of the immunity of the skin is further carried out by the electric stimulus.

12. A method of administrating a vaccine according to any one of claims 9 to 11, wherein the first administration of the vaccine is carried out under the condition that a pH of a drug solution existing in the vaccine containing layer is greater than or equal to 6.

13. A method of administrating a vaccine according to any one of claims 9 to 12, wherein the first administration of the vaccine is carried out under the condition that a pH of a drug solution existing in the vaccine containing layer is greater than or equal to 6, followed by the second or later administration of the vaccine is further carried out.

14. An apparatus for iontophoresis, wherein the drug product according to any one of claims 1 to 8 is used as an electrode.

15. An apparatus for iontophoresis according to claim 14, wherein the drug product is used as an electrode of the cathode side.

16. An apparatus for iontophoresis according to claim 14, wherein the drug product is used as an electrode of both the cathode side and the anode side.
